Europäisches Patentamt

European Patent Office

Office européen des brevets

(11) Veröffentlichungsnummer: **0 320 741**

**A1**

# EUROPÄISCHE PATENTANMELDUNG

(21) Anmeldenummer: 88120297.2

(51) Int. Cl.4: **A61B 6/00**

(22) Anmeldetag: 05.12.88

(30) Priorität: 18.12.87 DE 8716725 U

(43) Veröffentlichungstag der Anmeldung:
21.06.89 Patentblatt 89/25

(84) Benannte Vertragsstaaten:
BE DE FR GB

(71) Anmelder: **Siemens Aktiengesellschaft Berlin
und München
Wittelsbacherplatz 2
D-8000 München 2(DE)**

(72) Erfinder: **Hahn, Alfred, Dipl.-Ing.
Bunsenstrasse 64
D-8520 Erlangen(DE)**
Erfinder: **Raup, Jens-Peter, Dipl.-Ing.
Albert-Schweitzer-Strasse 24
D-8525 Uttenreuth(DE)**

(54) Röntgenuntersuchungseinrichtung zur wahlweisen Durchleitung oder Aufnahme eines Untersuchungsobjektes.

(57) Eine Röntgenuntersuchungseinrichtung zur wahlweisen Durchleuchtung oder Aufnahme ist mit einem in bezug auf ein Untersuchungsobjekt (2) verstellbaren C-Bogen (4) versehen, an dessen einem Ende ein Röntgenstrahler (5) und an dessen anderem Ende zur Durchleuchtung ein Röntgenbildverstärker (6) angeordnet ist, wobei zur Anfertigung einer Aufnahme ein Aufnahmegerät (10) dem Röntgenstrahler (5) gegenüberliegend positionierbar ist. Dabei ist vorgesehen, daß das Aufnahmegerät (10) mittels einer Tragvorrichtung (11) in bezug auf den C-Bogen (4) räumlich frei verstellbar gehalten ist, daß das Aufnahmegerät (10) mittels einer lösbaren Koppeleinrichtung (23) mit dem C-Bogen (4) verbindbar ist und daß die Koppeleinrichtung (23) derart ausgebildet ist, daß das Aufnahmegerät (10) den Bewegungen des C-Bogens (4) folgt, ohne seine Position relativ zu dem Röntgenstrahler (5) zu verändern.

FIG 1

## Röntgenuntersuchungseinrichtung zur wahlweisen Durchleuchtung oder Aufnahme eines Untersuchungsobjektes

Die Erfindung betrifft eine Röntgenuntersuchungseinrichtung zur wahlweisen Durchleuchtung oder Aufnahme eines Untersuchungsobjektes mit einem in bezug auf das Untersuchungsobjekt verstellbaren Röntgendiagnostikgerät, welches einen verstellbaren C-Bogen aufweist, an dessen einem Ende ein Röntgenstrahler und an dessen anderem Ende zur Durchleuchtung des Untersuchungsobjektes ein Röntgenbildverstärker angeordnet ist, wobei zur Anfertigung einer Aufnahme des Untersuchungsobjektes ein Aufnahmegerät dem Röntgenstrahler gegenüberliegend positionierbar ist.

Derartige Röntgenuntersuchungseinrichtungen bieten die Möglichkeit, von einem im Durchleuchtungsbetrieb erkennbaren diagnostisch relevanten Objekt eine Direktaufnahme anfertigen zu können.

Eine Röntgenuntersuchungseinrichtung der eingangs genannten Art ist aus der Werbedruckschrift "ANGIOSKOP A33" der Firma Siemens-Elema AB. Schweden, Best.-Nr.: 91003-M103-A727-02, bekannt. Dabei ist das Aufnahmegerät, es handelt sich um einen Blattfilmwechsler, an dem Röntgendiagnostikgerät angebracht, indem es mit dem Röntgenbildverstärker zu einer Einheit verbunden ist, die um eine Achse derart schwenkbar ist, daß wahlweise der Röntgenbildverstärker oder das Aufnahmegerät dem Röntgenstrahler gegenüberliegend positionierbar ist. Die bekannte Röntgenuntersuchungseinrichtung gestattet es somit, auf einfache Weise vom Durchleuchtungs- zum Aufnahmebetrieb überzugehen, nämlich durch geeignetes Schwenken der aus Röntgenbildverstärker und Aufnahmegerät gebildeten Einheit. Außerdem folgt das Aufnahmegerät beliebigen Bewegungen des Röntgendiagnostikgerätes, da es mit dessen Röntgenbildverstärker verbunden ist. Hieraus resultiert jedoch der Nachteil, daß im Falle der be kannten Röntgenuntersuchungseinrichtung der C-Bogen des Röntgendiagnostikgerätes nicht nur durch das Gewicht des Röntgenbildverstärkers und des Röntgenstrahlers, sondern außerdem durch das nicht unbeträchtliche Gewicht des Aufnahmegerätes belastet ist. Der C-Bogen und dessen Lagerung müssen demzufolge entsprechend massiv dimensioniert sein, was zu zusätzlichen Kosten führt. Außerdem schränkt die Einheit aus Röntgenbildverstärker und Aufnahmegerät infolge ihres erheblichen Raumbedarfes bei bestimmten Untersuchungen die Zugänglichkeit zu dem Untersuchungsobjekt ein.

Der Erfindung liegt die Aufgabe zugrunde, eine Röntgenuntersuchungseinrichtung der eingangs genannten Art so auszubilden, daß der C-Bogen des Röntgendiagnostikgerätes von dem Gewicht des Aufnahmegerätes entlastet und eine verbesserte Zugänglichkeit zu dem Untersuchungsobjekt gewährleistet ist, und dennoch sicherzustellen, daß ein einfacher Übergang vom Durchleuchtungs- zum Aufnahmebetrieb möglich ist und das Aufnahmegerät beliebigen Bewegungen des Röntgendiagnostikgerätes folgt.

Nach der Erfindung wird diese Aufgabe dadurch gelöst, daß das Aufnahmegerät mittels einer Tragvorrichtung in bezug auf das Röntgendiagnostikgerät räumlich frei verstellbar gehalten ist, daß das Aufnahmegerät mittels einer lösbaren Koppeleinrichtung mit dem Röntgendiagnostikgerät verbindbar ist und daß die Koppeleinrichtung derart ausgebildet ist, daß das Aufnahmegerät den Bewegungen des Röntgendiagnostikgerätes folgt, ohne seine Position relativ zu dem Röntgenstrahler zu verändern. Da für das Aufnahmegerät eine Tragvorrichtung vorgesehen ist, wird also der C-Bogen des Röntgendiagnostikgerätes von dem Gewicht des Aufnahmegerätes entlastet. Außerdem ergibt sich eine verbesserte Zugänglichkeit zu dem Untersuchungsobjekt, da das Aufnahmegerät nur dann mittels der lösbaren Koppeleinrichtung mit dem Röntgendiagnostikgerät verbunden werden muß, wenn es tatsächlich zur Anfertigung einer Aufnahme benötigt wird. Ein einfacher Übergang vom Durchleuchtungs- zum Aufnahmebetrieb ist dadurch sichergestellt, daß das Aufnahmegerät lediglich mittels der Koppeleinrichtung mit dem Röntgendiagnostikgerät verbunden werden muß. Außerdem ist die Koppeleinrichtung derart ausgebildet, daß das an der Tragvorrichtung frei verstellbar gehaltene Aufnahmegerät beliebigen Bewegungen des Röntgendiagnostikgerätes folgt.

Eine Variante der Erfindung sieht vor, daß die Tragvorrichtung derart ausgebildet ist, daß das Aufnahmegerät bei gelöster Koppeleinrichtung in eine Parkstellung außerhalb des Verstellbereiches des Röntgendiagnostikgerätes verstellbar ist. Es ist so sichergestellt, daß das von dem Röntgendiagnostikgerät getrennte Aufnahmegerät keine Behinderung für die Bewegungen des Röntgendiagnostikgerätes darstellt.

Wenn der C-Bogen um eine in der Ebene des C-Bogens verlaufende erste Achse und um eine senkrecht zu der Ebene des C-Bogens verlaufende zweite Achse schwenkbar ist, wobei sich die beiden Achsen in einem Isozentrum schneiden können, um das der C-Bogen schwenkbar ist und durch das der Zentralstrahl eines von dem Röntgenstrahler ausgehenden Röntgenstrahlenbündels verläuft, ist nach einer Ausführungsform der Erfindung vorgesehen, daß das Aufnahmegerät an der Tragvorrichtung um eine parallel zu der ersten Achse verlaufende dritte Achse und eine parallel zu der zweiten Achse verlaufende vierte Achse schwenkbar angebracht ist. Das Aufnahmegerät kann somit den angegebenen Bewegungen des Röntgendiagnostikgerätes bzw. des C-Bogens in der erforderlichen Weise folgen.

Nach einer Variante der Erfindung weist die Koppeleinrichtung eine Koppelstange auf, mittels derer das Aufnahmegerät starr mit dem Röntgendiagnostikgerät koppelbar ist, so daß sichergestellt ist, daß das Aufnahmegerät seine Position relativ zu dem Röntgenstrahler des Röntgendiagnostikgerätes beibehält.

Die Tragvorrichtung kann nach einer Variante der Erfindung einen Arm aufweisen, an dem das Aufnahmegerät angebracht ist, wobei der Arm mit der Tragvorrichtung um die dritte Achse schwenkbar und mit dem Aufnahmegerät in bezug auf diese starr verbunden ist, während das Aufnahmegerät um die vierte Achse schwenkbar mit dem Arm verbunden ist, der seinerseits in bezug auf diese starr mit der Tragvorrichtung verbunden ist. Es ist so auf konstruktiv einfache Weise sichergestellt, daß das Aufnahmegerät die erforderlichen Schwenkbewegungen bezüglich der dritten und der vierten Achse ausführen kann.

Eine Ausführungsform der Erfindung sieht vor, daß die Tragvorrichtung eine Gewichtsausgleichsvorrichtung für das Gewicht des Aufnahmegerätes aufweist, so daß es möglich ist, das Aufnahmegerät mit geringem Kraftaufwand an das Röntgendiagnostikgerät heranzufahren und mit diesem zu verbinden. Außerdem können Mittel vorgesehen sein, die das durch das Gewicht des Aufnahmegerätes in bezug auf die dritte und/oder vierte Achse jeweils erzeugte Drehmoment ausgleichen. In diesem Zusammenhang ergibt sich eine besonders einfache Ausführung der erfindungsgemäßen Röntgenuntersuchungseinrichtung, wenn die vierte Achse durch den Schwerpunkt des Aufnahmegerätes verläuft, da das Gewicht des Aufnahmegerätes bezüglich dieser Achse dann kein Drehmoment erzeugen kann.

Um die erforderliche freie räumliche Verstellbarkeit des Aufnahmegerätes sicherzustellen, ist nach einer Ausführungsform der Erfindung vorgesehen, daß die Tragvorrichtung ein Deckenstativ mit einer Teleskopsäule aufweist, wobei die Teleskopsäule an einem Wagen angebracht ist, der längs einer Schiene verfahrbar ist, die ihrerseits längs einer quer zu ihr verlaufenden Deckenschiene verfahrbar ist, wobei das Aufnahmegerät an dem freien Ende der Teleskopsäule angebracht ist. In diesem Falle ist es zweckmäßig, wenn der Arm der Tragvorrichtung am freien Ende der Teleskopsäule um die dritte Achse schwenkbar angebracht ist und eine solche Länge aufweist, daß der C-Bogen um die erste Achse in beiden Richtungen um jeweils wenigstens 15° schwenkbar ist, da dann mittels der erfindungsgemäßen Röntgenuntersuchungseinrichtung alle wesentlichen Untersuchungen durchgeführt werden können.

Ein Ausführungsbeispiel der Erfindung ist in den Fig. der beigefügten Zeichnungen dargestellt. Es zeigen:

Fig. 1 eine Seitenansicht einer erfindungsgemäßen Röntgenuntersuchungseinrichtung, und

Fig. 2 eine perspektivische Ansicht der Röntgenuntersuchungseinrichtung nach Fig. 1.

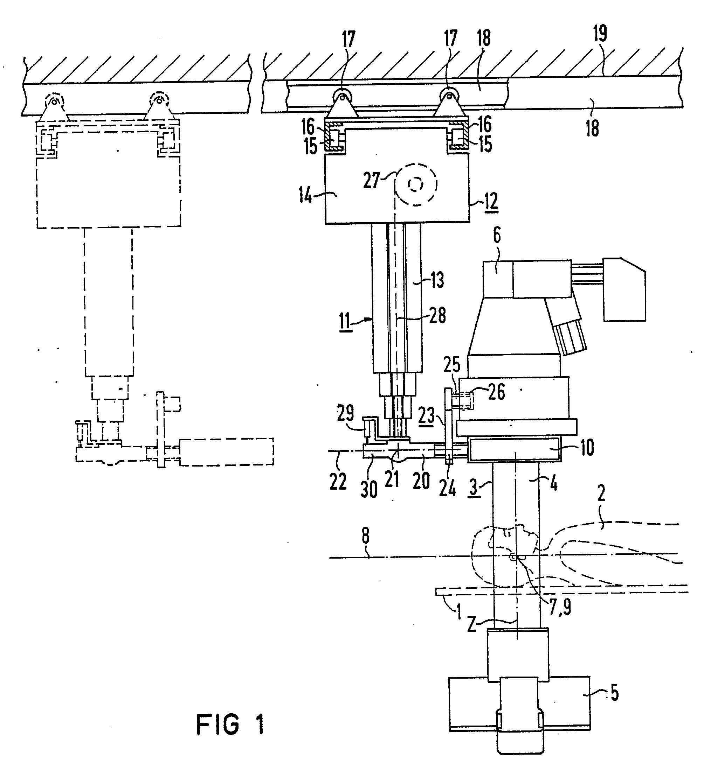

In den Fig. ist eine erfindungsgemäße Röntgenuntersuchungseinrichtung zur wahlweisen Durchleuchtung oder Aufnahme eines in Fig. 1 strichliert angedeuteten, auf einem Patientenlagerungstisch 1 liegenden Untersuchungsobjektes 2 dargestellt, die ein in bezug auf das Untersuchungsobjekt 2 verstellbares, insgesamt mit 3 bezeichnetes Röntgendiagnostikgerät aufweist. Dieses wiederum weist einen C-Bogen 4 auf, an dessen einem Ende ein Röntgenstrahler 5 und an dessen anderem Ende zur Durchleuchtung des Untersuchungsobjektes 2 ein dem Röntgenstrahler 5 gegenüberliegender Röntgenbildverstärker 6 angeordnet ist.

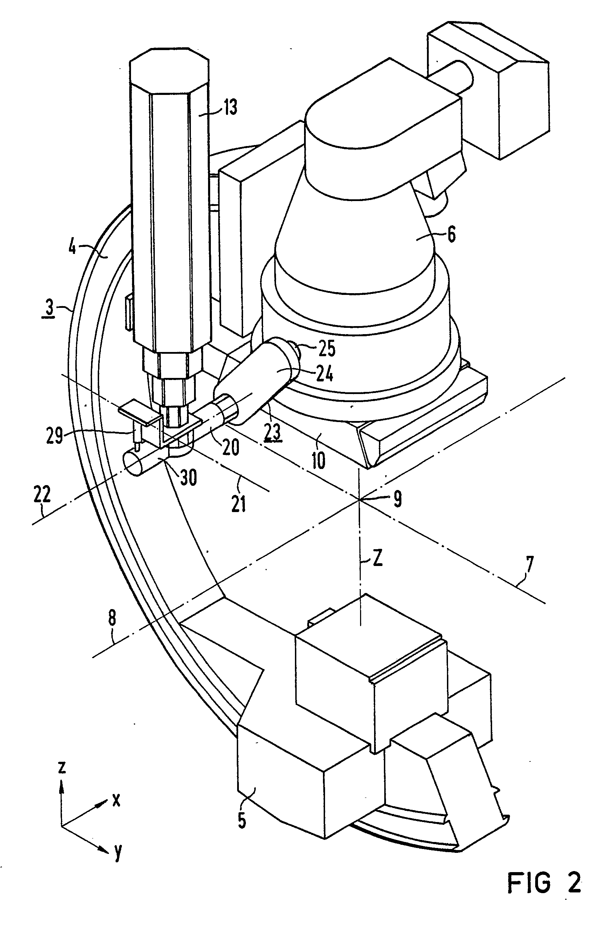

Der C-Bogen 4 ist an einer in den Fig. nicht dargestellten Halterung angebracht, und zwar derart, daß er um eine in der Ebene des C-Bogens 4 verlaufende erste Achse 7 und um eine senkrecht zu der Ebene des C-Bogens 4 verlaufende zweite Achse 8 schwenkbar ist. Außerdem ist der C-Bogen 4 in Richtung der drei Raumachsen x, y, z, die in Fig. 2 angedeutet sind, verstellbar. Die beiden Achsen 7 und 8 schneiden sich in einem Isozentrum 9, um das der C-Bogen 4 schwenkbar ist und durch das der strichpunktiert angedeutete Zentralstrahl Z eines von dem Röntgenstrahler 5 ausgehenden Röntgenstrahlenbündels verläuft. Zur Durchführung einer Untersuchung wird das Röntgendiagnostikgerät 3 relativ zu dem Untersuchungsobjekt 2 so positioniert, daß sich der diagnostisch relevante Bereich des Untersuchungsobjektes 2 im Isozentrum 9 befindet. Durch Schwenken des C-Bogens 4 um die Achsen 7 und 8 kann dann der diagnostisch relevante Bereich aus unterschiedlichen Richtungen durchstrahlt werden.

Um von dem diagnostisch relevanten Bereich des Untersuchungsobjektes 2, der mit Hilfe einer in den Fig. nicht dargestellten Röntgenfernseheinrichtung auf einem Monitor sichtbar gemacht werden kann, im Bedarfsfalle auch eine Röntgenaufnahme anfertigen zu können, ist ein Aufnahmegerät, nämlich ein Blattfilmwechsler 10, vorgesehen, der dem Röntgenstrahler 5 gegenüberliegend positionierbar ist.

Der Blattfilmwechsler 10 ist an einer insgesamt mit 11 bezeichneten Tragvorrichtung angebracht, die ein Deckenstativ 12 mit einer Teleskopsäule 13 aufweist. Diese ist gemäß Fig. 1 an einem Wagen 14 angebracht, der mit Hilfe von Rollen 15 längs zweier paralleler Schienen 16 verfahrbar ist. Diese sind ihrerseits mit Hilfe von Rollen 17 in zwei parallel zueinander verlaufenden Deckenschienen 18 verfahrbar, die an der Decke 19 des Untersuchungsraumes angebracht sind und quer zu den Schienen 16 verlaufen. Da der Blattfilmwechsler 10 an dem freien Ende der Teleskopsäule 13 angebracht ist, ist er mittels des Deckenstatives 12 in Richtung der drei Raumachsen x, y, z verstellbar.

Im einzelnen ist der Blattfilmwechsler 10 mittels eines zu der Tragvorrichtung 11 gehörigen Armes 20 an dem freien Ende der Teleskopsäule 13 angebracht. Der Arm 20 ist mittels eines nicht näher dargestellten Gelenkes um eine parallel zu der ersten Achse 7 verlaufende dritte Achse 21 schwenkbar mit dem freien Ende der Teleskopsäule 13 verbunden, während er bezüglich der Achse 21 starr mit dem Blattfilmwechsler 10 verbunden ist. Allerdings ist der Arm 20 mit dem Blattfilmwechsler 10 bezüglich einer parallel zu der zweiten Achse 8 verlaufenden vierten Achse 22 mittels eines ebenfalls nicht näher dargestellten Gelenkes schwenkbar verbunden. Bezüglich dieser Achse ist der Arm 20 mit der Teleskopsäule 13 starr verbunden. Der Blattfilmwechsler 10 ist somit mittels der Tragvorrichtung 11 nicht nur, wie bereits erwähnt, in Richtung der drei Raumachsen x, y, z verstellbar, sondern außerdem um die dritte und die vierte Achse 21, 22 schwenkbar und somit in bezug auf das Röntgendiagnostikgerät 3 frei verstellbar gehalten.

Die erfindungsgemäße Röntgendiagnostikeinrichtung weist außerdem eine lösbare Koppeleinrichtung 23 auf, mittels derer der Blattfilmwechsler 10 mit dem Röntgendiagnostikgerät 3 verbindbar ist. Im einzelnen umfaßt die Koppeleinrichtung 23 eine Koppelstange 24, die mit ihrem einen Ende fest mit dem Arm 20 verbunden ist und an ihrem anderen Ende einen Zapfen 25 aufweist, der in eine entsprechende Bohrung 26 einrastbar ist, die in dem Röntgenbildverstärker 6 des Röntgendiagnostikgerätes 3 angebracht ist. Der Blattfilmwechsler 10 ist somit mittels der Koppelstange 24 starr mit dem Röntgendiagnostikgerät 3 koppelbar. Dabei ist die Anordnung so getroffen, daß dann, wenn der Zapfen 25 der Koppelstange 24 in die Bohrung 26 eingerastet ist, der Blattfilmwechsler 10 in der zur Anfertigung einer Aufnahme erforderlichen Position dem Röntgenstrahler 5 gegenüberliegend angeordnet ist. Da der Blattfilmwechsler 10 einerseits mittels der Koppeleinrichtung 23 starr mit dem Röntgendiagnostikgerät 3 koppelbar ist und andererseits infolge der beschriebenen Ausbildung der Tragvorrichtung 11 in bezug auf das Röntgendiagnostikgerät 3 räumlich frei verstellbar ist, kann der mittels der Koppeleinrichtung 23 mit dem Röntgendiagnostikgerät 3 gekoppelte Blattfilmwechsler 10 den Bewegungen des Röntgendiagnostikgerätes 3 folgen, ohne seine Position relativ zu dem Röntgenstrahler 5 zu verändern. Dies gilt nicht nur für Schwenkbewegungen des C-Bogens 4 des Röntgendiagnostikgerätes 3 um die Achsen 7 und 8, sondern auch für Bewegungen des Röntgendiagnostikgerätes 3 in Richtung der drei Raumachsen x, y, z.

In dem Wagen 14 ist eine strichliert angedeutete Gewichtsausgleichsvorrichtung 27 angeordnet, von der ein Seil 28 zur dem freien Ende der Teleskopsäule 13 führt, über das die Gewichtsausgleichsvorrichtung 27 eine Kraft ausübt, die dem Gewicht des Blattfilmwechslers 10 entspricht. Außerdem ist am freien Ende der Teleskopsäule 13 eine Gasfeder 29 angebracht, die auf einen von dem Blattfilmwechsler 10 aus gesehen jenseits der dritten Achse 21 befindlichen Abschnitt 30 des Armes 20 eine Kraft ausübt, die das durch das Gewicht des Blattfilmwechslers 10 in bezug auf die dritte Achse 21 erzeugte Drehmoment ausgleicht. Die vierte Achse 22 verläuft durch den Schwerpunkt des Blattfilmwechslers 10, so daß dessen Gewicht bezüglich der vierten Achse 22 kein Drehmoment ausüben kann.

Es wird somit deutlich, daß das Röntgendiagonstikgerät 3 bzw. dessen C-Bogen 4 völlig von dem Gewicht des Blattfilmwechslers 10 entlastet ist. Bei einer Verstellung des Röntgendiagnostikgerätes 3 mit angekoppeltem Blattfilmwechsler 10 müssen zur Verstellung des Blattfilmwechslers 10 also nur diejenigen Kräfte aufgebracht werden, die zur Überwindung der Massenträgheit und etwaiger Reibungskräfte erforderlich sind. Außerdem kann der Blattfilmwechsler 10 mit geringem Kraftaufwand von dem Bedienungspersonal an das Röntgendiagnostikgerät 3 herangefahren und mit diesem gekoppelt bzw. von diesem getrennt werden.

Der Arm 20 der Tragvorrichtung 11 weist übrigens eine solche Länge auf, daß der C-Bogen um die erste Achse 7 in beiden Richtungen um jeweils wenigstens 15° schwenkbar ist.

Die Schienen 16 und die Deckenschienen 18 weisen eine solche Länge auf, daß der Blattfilmwechsler 10 bei gelöster Koppeleinrichtung 23 in eine Parkstellung außerhalb des Verstellbereiches des Röntgendiagnostikgerätes 3 verstellbar ist, wie dies in Fig. 1 strichliert angedeutet ist.

**Ansprüche**

1. Röntgenuntersuchungseinrichtung zur wahlweisen Durchleuchtung oder Aufnahme eines Untersuchungsobjektes (2), mit einem in bezug auf das Untersuchungsobjekt (2) verstellbaren Röntgendiagnostikgerät (3), welches einen verstellbaren C-Bogen (4) aufweist, an dessen einem Ende ein Röntgenstrahler (5) und an dessen anderem Ende zur Durchleuchtung des Untersuchungsobjektes (2) ein Röntgenbildverstärker (6) angeordnet ist, wobei zur Anfertigung einer Aufnahme des Untersuchungsobjektes (2) ein Aufnahmegerät (10) dem Röntgenstrahler (5) gegenüberliegend positionierbar ist, **dadurch gekennzeichnet, daß** das Aufnahmegerät (10) mittels einer Tragvorrichtung (11) in bezug auf das Röntgendiagnostikgerät (3) räumlich frei verstellbar gehalten ist, daß das Aufnahmegerät (10) mittels einer lösbaren Koppeleinrichtung (23) mit dem Röntgendiagnostikgerät (3) verbindbar ist und daß die Koppeleinrichtung (23) derart ausgebildet ist, daß das Aufnahmegerät (10) den Bewegungen des Röntgendiagnostikgerätes (3) folgt, ohne seine Position relativ zu dem Röntgenstrahler (5) zu verändern.

2. Röntgenuntersuchungseinrichtung nach Anspruch 1, **dadurch gekennzeichnet, daß** die Tragvorrichtung (11) derart ausgebildet ist, daß das Aufnahmegerät (10) bei gelöster Koppeleinrichtung (23) in eine Parkstellung außerhalb des Verstellbereiches des Röntgendiagnostikgerätes (3) verstellbar ist.

3. Röntgenuntersuchungseinrichtung nach Anspruch 1 oder 2, deren C-Bogen (4) um eine in der Ebene des C-Bogens (4) verlaufende erste Achse (7) und um eine senkrecht zu der Ebene des C-Bogens (4) verlaufende zweite Achse (8) schwenkbar ist, wobei sich vorzugsweise die beiden Achsen (7, 8) in einem Isozentrum (9) schneiden, um das der C-Bogen (4) schwenkbar ist und durch das der Zentralstrahl (Z) eines von dem Röntgenstrahler (5) ausgehenden Röntgenstrahlenbündels verläuft, **dadurch gekennzeichnet, daß** das Aufnahmegerät (10) an der Tragvorrichtung (11) um eine parallel zu der ersten Achse (7) verlaufende dritte Achse (21) und eine parallel zu der zweiten Achse (8) verlaufende vierte Achse (22) schwenkbar angebracht ist.

4. Röntgenuntersuchungseinrichtung nach einem der Ansprüche 1 bis 3, **dadurch gekennzeichnet, daß** die Koppeleinrichtung (23) eine Koppelstange (24) aufweist, mittels derer das Aufnahmgerät (10) starr mit dem Röntgendiagnostikgerät (3) koppelbar ist.

5. Röntgenuntersuchungseinrichtung nach Anspruch 3 oder 4, **dadurch gekennzeichnet, daß** die Tragvorrichtung (11) einen Arm (20) aufweist, an dem das Aufnahmgerät (10) angebracht ist, wobei der Arm (20) mit der Tragvorrichtung (11) um die dritte Achse (21) schwenkbar und mit dem Aufnahmegerät (10) in bezug auf diese starr verbunden ist, während das Aufnahmegerät (10) um die vierte Achse (22) schwenkbar mit dem Arm (20) verbunden ist, der seinerseits in bezug auf diese starr mit der Tragvorrichtung (11) verbunden ist.

6. Röntgenuntersuchungseinrichtung nach einem der Ansprüche 1 bis 5, **dadurch gekennzeichnet, daß** die Tragvorrichtung (11) eine Gewichtsausgleichsvorrichtung (27) für das Gewicht des Aufnahmegerätes (10) aufweist.

7. Röntgenuntersuchungseinrichtung nach einem der Ansprüche 3 bis 6, **dadurch gekennzeichnet, daß** Mittel (29, 30) vorgesehen sind, die das durch das Gewicht des Aufnahmegerätes (10) in bezug auf die dritte und/oder vierte Achse (21 bzw. 22) jeweils erzeugte Drehmoment ausgleichen.

8. Röntgenuntersuchungseinrichtung nach einem der Ansprüche 3 bis 7, **dadurch gekennzeichnet, daß** die vierte Achse (22) durch den Schwerpunkt des Aufnahmegerätes (10) verläuft.

9. Röntgenuntersuchungseinrichtung nach einem der Ansprüche 1 bis 8, **dadurch gekennzeichnet, daß** die Tragvorrichtung (11) ein Deckenstativ (12) mit einer Teleskopsäule (13) aufweist, wobei die Teleskopsäule (13) an einem Wagen (14) angebracht ist, der längs einer Schiene (16) verfahrbar ist, die ihrerseits längs einer quer zu ihr verlaufenden Deckenschiene (18) verfahrbar ist, wobei das Aufnahmegerät (10) an dem freien Ende der Teleskopsäule (13) angebracht ist.

10. Röntgenuntersuchungseinrichtung nach den Ansprüchen 5 und 9, **dadurch gekennzeichnet, daß** der Arm (20) der Tragvorrichtung (11) am freien Ende der Teleskopsäule (13) um die dritte Achse (21) schwenkbar angebracht ist und eine solche Länge aufweist, daß der C-Bogen (4) um die erste Achse (7) in beiden Richtungen um jeweils wenigstens 15° schwenkbar ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 922 960 (NRT) * Seite 7, Zeilen 9-32; Seite 8, Zeilen 10-30; Figuren 1-3 * | 1 | A 61 B 6/00 |
| A | --- | 3 | |
| Y | US-A-4 024 401 (S. BERNSTEIN et al.) * Zusammenfassung; Spalte 2, Zeilen 42-47,59-65; Spalte 3, Zeilen 49-59; Spalte 4, Zeilen 23-49; Spalte 5, Zeilen 14-56; Figuren 1-5 * | 1 | |
| A | --- | 2,4,6,9 ,10 | |
| A | PATENT ABSTRACTS OF JAPAN, Band 6, Nr. 38 (P-105)[916], 9. März 1982; & JP-A-56 155 937 (SHIMAZU SEISAKUSHO K.K.) 02-12-1981 * Zusammenfassung * | 1,3,9 | |
| A | EP-A-0 205 878 (SIEMENS AG) * Zusammenfassung; Spalte 2, Zeilen 10-55; Figuren 1,2 * | 1,3,10 | RECHERCHIERTE SACHGEBIETE (Int. Cl.4) A 61 B |

Der vorliegende Recherchenbericht wurde für alle Patentansprüche erstellt

| Recherchenort | Abschlußdatum der Recherche | Prüfer |
|---|---|---|
| DEN HAAG | 10-03-1989 | RIEB K.D. |